(19) Europäisches Patentamt
European Patent Office
Office européen des brevets

(11) EP 1 882 709 B1

(12) **FASCICULE DE BREVET EUROPEEN**

(45) Date de publication et mention de la délivrance du brevet:
**25.11.2009 Bulletin 2009/48**

(51) Int Cl.:
***C08F 293/00*** *(2006.01)* ***A61K 8/81*** *(2006.01)*
***A61K 8/90*** *(2006.01)*

(21) Numéro de dépôt: **07108112.9**

(22) Date de dépôt: **14.05.2007**

(54) **Polymères séquencés, et leur procédé de préparation**

Blockpolymere und Verfahren zu ihrer Herstellung

Block copolymers and method for their preparation

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **27.07.2006 FR 0653144**

(43) Date de publication de la demande:
**30.01.2008 Bulletin 2008/05**

(73) Titulaire: **L'Oréal**
**75008 Paris (FR)**

(72) Inventeurs:
- **Farcet, Céline**
**75012, Paris (FR)**
- **Lion, Bertrand**
**75012, Paris (FR)**

(74) Mandataire: **Chantraine, Sylvie Hélène**
**L'ORÉAL,**
**River Plaza,**
**25-29 Quai Aulagnier**
**92600 Asnieres-sur-Seine (FR)**

(56) Documents cités:
**EP-A- 1 411 069** **EP-A- 1 421 928**
**EP-A- 1 518 534** **EP-A- 1 518 535**
**US-A1- 2005 095 213**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 882 709 B1

## Description

La présente invention a trait à de nouveaux polymères séquencés de structure spécifique. Ces polymères comprennent au moins une première séquence et au moins une deuxième séquence, les deux séquences étant avantageusement telles que l'une des séquences a une température de transition vitreuse supérieure à 20°C (séquence dite rigide), l'autre séquence a une température de transition vitreuse inférieure ou égale à 20 °C (séquence dite souple).

Des polymères de structure proche sont décrits dans la demande EP1411069. La présente invention se fixe pour objectif d'améliorer les propriétés des polymères décrits dans ce document, pour obtenir des compositions cosmétiques dont la tenue est améliorée à niveau de brillance équivalent.

Ce but est atteint, conformément à la présente invention, grâce à un polymère séquencé, comprenant au moins une première séquence et au moins une deuxième séquence,
caractérisé en ce que la première séquence est obtenue à partir d'au moins un monomère acrylate de formule $CH_2$ = CH-$COOR_2$ dans laquelle $R_2$ représente un groupe cycloalkyle $C_4$ à $C_{12}$et d'au moins un monomère méthacrylate de formule $CH_2$ = $C(CH_3)$-$COOR_2$ dans laquelle $R'_2$ représente un groupe cycloalkyle $C_4$ à $C_{12}$,
et caractérisé en ce que la deuxième séquence est obtenue à partir d'un monomère acide acrylique et d'au moins un monomère de transition vitreuse inférieure ou égale à 20°C.

La demande EP1411069 décrit la possibilité de préparer des polymères séquencés à partir de monomère acrylate ou de monomère méthacrylate. Ce document décrit également d'une manière générale la possibilité d'incorporer des monomères acide acrylique. Cependant, ce document ne décrit pas la combinaison de caractéristiques revendiquées à savoir le mélange d'un monomère acrylate et d'un monomère méthacrylate du même alcool dans la séquence rigide et la présence d'acide acrylique dans la séquence souple.
En outre, ce document n'incite pas l'homme du métier à modifier la nature des monomères pour obtenir un copolymère qui permette d'améliorer les propriétés de tenue d'une composition cosmétique brillante. Les polymères décrits dans ce document doivent être associés pour obtenir une composition cosmétique dotée de ces deux propriétés à la fois.

Ainsi l'invention a pour objet un nouveau polymère séquencé. Elle a encore pour objet une composition cosmétique contenant un tel polymère.

Un autre objet de l'invention est aussi un procédé cosmétique de maquillage ou de soin des matières kératiniques comprenant l'application sur les matières kératiniques, d'une composition cosmétique selon l'invention.

L'invention se rapporte également à un procédé de préparation du polymère séquencé, ainsi qu'au polymère susceptible d'être obtenu par ce procédé.

L'invention a encore à l'utilisation du polymère selon l'invention dans une composition cosmétique, comme agent pour améliorer la tenue de ladite composition tout en maintenant sa brillance.

L'invention se rapporte également à l'utilisation du polymère selon l'invention dans une composition présentant des propriétés de tenue améliorées et une bonne brillance.

Le polymère séquencé selon l'invention comprend au moins une première séquence et au moins une deuxième séquence.
Par "au moins" une séquence, on entend une ou plusieurs séquences.
Par polymère "séquencé", on entend un polymère comprenant au moins 2 séquences distinctes, de préférence au moins 3 séquences distinctes.

La première séquence et la deuxième séquence du polymère de l'invention peuvent être avantageusement incompatibles l'une avec l'autre.
Par "séquences incompatibles l'une avec l'autre", on entend que le mélange formé par un polymère correspondant à la première séquence et par un polymère correspondant à la deuxième séquence, n'est pas miscible dans le solvant de polymérisation, majoritaire en poids, du polymère séquencé, à température ambiante (25 °C) et pression atmosphérique ($10^5$ Pa), pour une teneur du mélange desdits polymères supérieure ou égale à 5 % en poids, par rapport au poids total du mélange desdits polymères et dudit solvant de polymérisation, étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.
Bien entendu, dans le cas d'une polymérisation réalisée dans un solvant unique, ce dernier est le solvant majoritaire.

Le polymère séquencé selon l'invention comprend au moins une première séquence et au moins une deuxième séquence.
Lesdites première et deuxième séquences peuvent être avantageusement reliées entre elles par un segment intermédiaire comprenant au moins un monomère constitutif de la première séquence et au moins un monomère constitutif de la deuxième séquence.

Le segment intermédiaire est une séquence comprenant au moins un monomère constitutif de la première séquence et au moins un monomère constitutif de la deuxième séquence du polymère permet de "compatibiliser" ces séquences.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Le polymère séquencé selon l'invention est avantageusement un polymère éthylénique séquencé filmogène.

Par polymère "éthylénique", on entend un polymère obtenu par polymérisation de monomères comprenant une insaturation éthylénique.

Par polymère "filmogène", on entend un polymère apte à former à lui seul ou en présence d'un agent auxiliaire de filmification, un film continu et adhérent sur un support, notamment sur les matières kératiniques.

De façon préférentielle, le polymère selon l'invention ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25 °C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

> On prépare un film de polymère par coulage d'une solution du polymère dans une matrice téflonnée puis séchage pendant 7 jours dans une ambiance contrôlée à 23±5°C et 50±10 % d'humidité relative.
> On obtient alors un film d'environ 100 µm d'épaisseur dans lequel sont découpées des éprouvettes rectangulaires (par exemple à l'emporte-pièce) d'une largeur de 15 mm et d'une longueur de 80 mm.
> On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure après 2 heures le taux d'allongement résiduel de l'éprouvette en pourcentage ($\varepsilon_{2h}$), 2 heures après retour à la contrainte charge nulle.

La recouvrance retardée en % ($R_{2h}$) est donnée par la formule ci-après:

$$R_{2h} = (\varepsilon_{max} - \varepsilon_{2h})/ \varepsilon_{max}) \times 100$$

A titre purement indicatif, un polymère selon un mode de réalisation de l'invention possède de préférence une recouvrance instantanée $R_i$ de 10% et une recouvrance retardée $R_{2h}$ de 30%.

L'indice de polydispersité du polymère de l'invention est avantageusement supérieur à 2.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère selon l'invention est de préférence inférieure ou égale à 300 000, elle va par exemple de 35 000 à 200 000, et mieux de 45 000 à 150 000 g/mol.

La masse moyenne en nombre (Mn) du polymère selon l'invention est de préférence inférieure ou égale à 70 000, elle va par exemple de 10 000 à 60 000, et mieux de 12 000 à 50 000 g/mol.

De préférence, l'indice de polydispersité du polymère selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

Le polymère séquencé de l'invention comprend au moins une première séquence et au moins une deuxième séquence.

La première séquence est avantageusement obtenue à partir d'au moins un monomère acrylate de formule $CH_2 = CH-COOR_2$ et d'au moins un monomère méthacrylate de formule $CH_2 = C(CH_3)-COOR_2$ dans laquelle $R_2$ représente un groupe cycloalkyle $C_4$ à $C_{12}$. Les monomères et leurs proportions sont de préférence choisis de telle sorte que la température de transition vitreuse de la première séquence est supérieure à 20°C.

La deuxième séquence est avantageusement obtenue à partir d'un monomère acide acrylique et d'au moins un monomère de transition vitreuse inférieure ou égale à 20°C.

Les monomères et leurs proportions sont de préférence choisis de telle sorte que la température de transition vitreuse de la deuxième séquence est inférieure ou égale à 20 °C.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i) \ ,$$

$\overline{\omega}_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30 °C.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>Première Séquence</u>

La première séquence a de préférence une Tg supérieure à 20°C par exemple une Tg allant de 20 à 170°C, de préférence supérieure ou égale à 50°C, allant par exemple de 50 °C à 160 °C, notamment allant de 90 °C à 130 °C.

Selon un mode de mise en oeuvre, la première séquence est obtenue à partir d'au moins un monomère acrylate de formule $CH_2 = CH-COOR_2$ dans laquelle $R_2$ représente un groupe cycloalkyle $C_4$ à $C_{12}$, et d'au moins un monomère méthacrylate de formule $CH_2 = C(CH_3)-COOR'_2$ dans laquelle $R'_2$ représente un groupe cycloalkyle $C_4$ à $C_{12}$.

La première séquence peut être obtenue exclusivement à partir dudit monomère acrylate et dudit monomère méthacrylate.

Le monomère acrylate et le monomère méthacrylate sont de préférence dans des propositions massiques comprises entre 30 :70 et 70 :30, de préférence entre 40 :50 et 50 :40, notamment de l'ordre de 50 :50.

La proportion de la première séquence va avantageusement de 20 à 90% en poids du polymère, mieux de 30 à 80% et encore mieux de 60 à 80%.

Selon un mode de mise en oeuvre, la première séquence est obtenue par polymérisation du méthacrylate d'isobornyle et de l'acrylate d'isobornyle.

La première séquence peut en outre comprendre :

- de l'acide (méth)acrylique, de préférence de l'acide acrylique,
- de l'acrylate de tertiobutyle
- les méthacrylates de formule $CH_2 = C(CH_3)\text{-}COOR$,

dans laquelle $R_1$ représente un groupe alkyle non substitué, linéaire ou ramifié, contenant de 1 à 4 atomes de carbone, tel qu'un groupe méthyle, éthyle, propyle ou isobutyle,

- les (méth)acrylamides de formule :

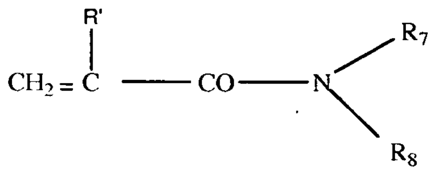

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

Deuxième séquence

La deuxième séquence a avantageusement une température de transition vitreuse Tg inférieure ou égale à 20 °C a par exemple une Tg allant de -100 à 20 °C, de préférence inférieure ou égale à 15°C, notamment allant de -80°C à 15°C et mieux inférieure ou égale à 10°C, par exemple allant de - 100 °C à 10 °C, notamment allant de -30°C à 10 °C.

La deuxième séquence est obtenue à partir d'un monomère acide acrylique et d'un autre monomère ayant une Tg inférieure ou égale à 20°C.

Le monomère ayant une Tg inférieure ou égale à 20°C est, de préférence, choisi parmi les monomères suivants:

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-}CO\text{-}O\text{-}CH = CH_2$
  où $R_5$ représente un groupe alkyle en $C_4$ à $C_{12}$ linéaire ou ramifié ;

- les éthers d'alcool vinylique et d'alcool en $C_4$ à $C_{12}$,

- les N-alkyl en $C_4$ à $C_{12}$ acrylamides, tels que le N-octylacrylamide,

- et leurs mélanges.

Les monomères ayant une Tg inférieure ou égale à 20°C préférés sont l'acrylate d'isobutyle, l'acrylate d'éthyl-2 hexyle ou leurs mélanges en toute proportions.

Chacune des première et deuxième séquences peut contenir en proportion minoritaire au moins un monomère constitutif de l'autre séquence.

Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé (s) un ou plusieurs hétéroatomes choisis parmi O, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène.

Le monomère additionnel peut représenter 0,5 à 30% en poids du poids du polymère. Selon un mode de mise en oeuvre, le polymère de l'invention ne contient pas de monomère additionnel. De préférence, le polymère de l'invention comprend au moins des monomères acrylate d'isobornyle et méthacrylate d'isobornyle dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence et des monomères acrylate d'isobutyle et acide acrylique dans la deuxième séquence.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la première séquence représentant 70% en poids du polymère.

De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la séquence de Tg supérieure à 20°C représentant 70% en poids du polymère, et l'acide acrylique représentant 5% en poids du polymère.

L'invention a également pour objet un procédé de préparation d'un polymère, consistant à mélanger, dans un même réacteur, un solvant de polymérisation, un amorceur, un monomère acide acrylique, au moins un monomère de transition vitreuse inférieure ou égale à 20°C, au moins un monomère acrylate de formule $CH_2 = CH\text{-}COOR_2$ dans laquelle $R_2$ représente un groupe cycloalkyle $C_4$ à $C_{12}$, et au moins un monomère méthacrylate de formule $CH_2 = C(CH_3)\text{-}COOR'_2$ dans laquelle $R'_2$ représente un groupe cycloalkyle $C_4$ à $C_{12}$, selon la séquence d'étape suivante :

- On verse dans le réacteur, une partie du solvant de polymérisation et une partie de l'amorceur, mélange que l'on chauffe à une température de réaction comprise entre 60 et 120°C,
- on verse ensuite, en une première coulée, ledit au moins monomère acrylate de formule $CH_2 = CH\text{-}COOR_2$ et ledit au moins monomère méthacrylate de formule $CH_2 = C(CH_3)\text{-}COOR'_2$ que l'on laisse à réagir pendant une durée T correspondant à un taux de conversion desdits monomères de 90% maximum,
- on verse ensuite dans le réacteur, en une deuxième coulée, à nouveau de l'amorceur de polymérisation, le monomère acide acrylique et ledit au moins monomère de transition vitreuse inférieure ou égale à 20°C, qu'on laisse réagir pendant une durée T' au bout de laquelle le taux de conversion desdits monomères atteint un plateau,
- on ramène le mélange réactionnel à température ambiante.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Selon un autre mode de mise en oeuvre, l'invention a pour objet un procédé de préparation d'un polymère, consistant à mélanger, dans un même réacteur, un solvant de polymérisation, un amorceur, un monomère acide acrylique, au moins un monomère de transition vitreuse inférieure ou égale à 20°C, au moins un monomère acrylate de formule $CH_2 = CH\text{-}COOR_2$ dans laquelle $R_2$ représente un groupe cycloalkyle $C_4$ à $C_{12}$, et au moins un monomère méthacrylate de formule $CH_2 = C(CH_3)\text{-}COOR'_2$ dans laquelle $R'_2$ représente un groupe cycloalkyle $C_4$ à $C_{12}$, selon la séquence d'étape suivante :

- On verse dans le réacteur, une partie du solvant de polymérisation et une partie de l'amorceur, mélange que l'on chauffe à une température de réaction comprise entre 60 et 120°C,
- on verse ensuite, en une première coulée, le monomère acide acrylique et ledit au moins monomère de transition vitreuse inférieure ou égale à 20°C que l'on laisse à réagir pendant une durée T correspondant à un taux de conversion desdits monomères de 90% maximum,
- on verse ensuite dans le réacteur, en une deuxième coulée, à nouveau de l'amorceur de polymérisation, ledit au moins monomère acrylate de formule $CH_2 = CH\text{-}COOR_2$ et ledit au moins monomère méthacrylate de formule $CH_2 = C(CH_3)\text{-}COOR'_2$, qu'on laisse réagir pendant une durée T' au bout de laquelle le taux de conversion desdits monomères atteint un plateau,
- on ramène le mélange réactionnel à température ambiante.

La température de polymérisation est de préférence de l'ordre de 90°C.

La durée de réaction après la deuxième coulée est de préférence comprise enter 3 et 6 heures.

Les monomères mis en oeuvre dans le cadre de ce procédé, ainsi que leurs proportions peuvent être ceux et celles décrites précédemment.

L'invention a également pour objet le polymère susceptible d'être obtenu par le procédé décrit précédemment.

L'invention concerne également des compositions cosmétiques comprenant au moins un polymère de structure spécifique, tel qu'il a été décrit ci-dessus.

Généralement, ces compositions contiennent de 0,1 à 60% en poids en matière active (ou matière séche) du polymère selon l'invention ou du polymère susceptible d'être obtenu par le procédé de l'invention, de préférence de 0,5 à 50 % en poids, et de préférence encore de 1 à 40% en poids.

Ces compositions cosmétiques, selon l'invention, comprennent, outre lesdits polymères, un milieu physiologiquement acceptable, c'est-à-dire un milieu compatible avec les matières kératiniques, comme la peau, les cheveux, les cils, les sourcils et les ongles.

Ledit milieu, physiologiquement acceptable, comprend généralement un solvant approprié, physiologiquement acceptable.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition peut comprendre, outre le polymère séquencé décrit précédemment selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer :

les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.
Ces huiles peuvent être présentes en une teneur allant de 0,01 à 90 %, et mieux de 0,1 à 85 % en poids, par rapport au poids total de la composition.

Par corps gras pâteux, on entend un produit visqueux contenant une fraction liquide et une fraction solide. Par « corps gras pâteux » au sens de l'invention, on entend des corps gras ayant un point de fusion allant de 20 à 55°C, de préférence 25 à 45°C, et/ou une viscosité à 40°C allant de 0,1 à 40 Pa.s (1 à 400 poises), de préférence 0,5 à 25 Pa.s, mesurée au Contraves TV ou Rhéomat 80. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.
Les valeurs de point de fusion correspondent, selon l'invention, au pic de fusion mesurée par la méthode "Differential Scanning Calorimetry" avec une montée en température de 5 ou 10 °C/min.

On peut utiliser un ou plusieurs corps gras pâteux. De préférence, ces corps gras sont des composés hydrocarbonés (contenant principalement des atomes de carbone et d'hydrogène et éventuellement des groupements ester), éventuellement de type polymérique; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40°C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly (12-hydroxystéarique) et leurs mélanges. Comme triglycérides d'origine végétale, on peut utiliser les dérivés d'huile de ricin hydrogénée, tels que le "THIXINR" de Rheox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503 et DC25514, et leurs mélanges.

Le ou les corps gras pâteux peuvent être présents à raison de 0,5 à 60% en poids, par rapport au poids total de la composition, de préférence à raison de 2-45% en poids et encore plus préférentiellement à raison de 5-30% en poids, dans la composition.

La composition selon l'invention peut également comprendre un ou plusieurs solvants organiques, cosmétiquement acceptables (tolérance, toxicologie et toucher acceptables). Ces solvants peuvent être généralement présents en une teneur allant de 0 à 90 %, de préférence de 0,1 à 90%, de préférence encore de 10 à 90% en poids, par rapport au poids total de la composition, et mieux de 30 à 90 %.

Comme solvants utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que

l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

La cire peut également présenter une dureté allant de 0,05 MPa à 15 MPa, et de préférence allant de 6 MPa à 15 MPa . La dureté est déterminée par la mesure de la force en compression mesurée à 20°C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre en outre une ou plusieurs charges, notamment en

une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.
Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara pour cheveux).

La composition selon l'invention peut également être un produit de soin de la peau du corps et du visage, notamment un produit solaire ou de coloration de la peau (tel qu'un autobronzant).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage.

L'exemple qui suit illustre de manière non limitative les compositions comprenant le polymère selon l'invention.

**Exemple 1 : Préparation d'un polymère de poly(acrylate d'isobornyle/méthacrylate d'isobornyle /acrylate isobutyle/acide acrylique)**

300 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g de méthacrylate d'isobornyle (fabriqué par Arkema), 105 g d'acrylate d'isobornyle (fabriqué par Arkema) et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 75 g d'acrylate d'isobutyle (fabriqué par Fluka), 15 g d'acide acrylique et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence rigide poly(acrylate d'isobornyle/méthacrylate

d'isobornyle) ayant une Tg de 110°C, une deuxième séquence souple poly(acrylate d'isobutyle/acide acrylique) ayant une Tg de - 9°C et une séquence intermédiaire qui est un polymère statistique acrylate d'isobornyle/méthacrylate d'isobornyle /acrylate d'isobutyle/acide acrylique.

**Exemples de polymère comparatif , exemples de polymère 2 et 3 :**

L'exemple comparatif est préparé selon l'enseignement de l'exemple 9 de la demande EP1411069 : poly (acrylate d'isobornyle/méthacrylate d'isobornyle /acrylate d'isobutyle). Le mode opératoire est rappelé ci-après :
100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobornyle) ayant une Tg de 128°C, une deuxième séquence polyacrylate d'isobutyle ayant une Tg de - 20°C et une séquence intermédiaire qui est un polymère statistique acrylate d'isobornyle/méthacrylate d'isobornyle/acrylate d'isobutyle.
Les exemples 2 et 3 de l'invention sont préparés selon le mode opératoire décrit dans l'exemple 1.
Leur composition est résumée dans le tableau ci-dessous.

| Exemples | Séquence de Tg > 20°C (wt.%) | | Séquence de Tg ≤ 20°C (wt.%) | |
|---|---|---|---|---|
| | Ac. Isobor. | Méth. Isobor. | Ac. Isobu. | AA |
| Comparatif | 35 | 35 | 30 | |
| Exemple 1 | 35 | 35 | 25 | 5 |
| Exemple 2 | 25 | 25 | 45 | 5 |
| Exemple 3 | 30 | 30 | 35 | 5 |
| Ac. Isobor. = acrylate d'isobornyle<br>Méth. Isobor. = méthacrylate d'isobornyle<br>Ac. Isobu. = acrylate d'isobutyle<br>AA = acide acrylique | | | | |

Tenue à l'huile des polymères des exemples:

Elle est mesurée avec une goutte d'huile d'olive mise sur un film de polymère sec.
Un film de polymère est réalisé à partir d'une solution de 20% dans l'isododécane, 0.5 ml est étalé sur une plaque de verre de 2.5*7.5 cm et laissé sécher à température ambiante pendant 24 heures. Ensuite, 1 ml d'huile d'olive est étalé sur le film de polymère. Après le temps voulu (1 heure ou 24 heures), l'excès d'huile est essuyé du film et ce dernier est pesé pour déterminer sa prise en masse.
L'exemple comparatif est collant après 1h ; on observe 5.5% de prise de masse après 24h, tandis que l'exemple 3 est non collant après 1 h ; sa prise de masse n'est que de 1 % après 24h.
La prise en masse et le collant traduisent la sensibilité du polymère à l'huile d'olive. Plus elle est importante, plus le dépôt sera facilement altéré lors des repas et la tenue sera moins bonne. La tenue du polymère de l'exemple 1 est supérieure à celle de l'exemple comparatif.

Brillance mesurée au brillancemètre sur un dépôt sec de polymère :

La brillance peut être mesurée à l'aide d'un brillancemètre, de manière conventionnelle par la méthode suivante.
Sur une carte de contraste de marque LENETA et de référence FORM 1A PENOPAC, on étale une couche de 50 µm d'épaisseur d'une solution du polymère à 50% dans le solvant de synthèse (isododécane) à l'aide d'un étaleur automatique. La couche recouvre au moins le fond noir de la carte. On laisse sécher le dépôt 24 heures à une température de 25°C, puis on procède à la mesure de la brillance à 20° sur le fond noir à l'aide d'un brillancemètre de marque DR

LANGE, REF03.

La brillance à 20 ° des polymères des exemples 1, 2 et 3 sont égales respectivement à 80, 74 et 75, tandis que la brillance de l'exemple comparatif est de 71.

On procède à la mesure de la brillance à 60° comme précédemment.
La brillance à 60 ° des polymères des exemples 1, 2 et 3 sont égales respectivement à 90, 87 et 87, tandis que la brillance de l'exemple comparatif est de 86.

**Exemple de gloss liquide contenant le polymère de l'exemple 1**

Le mode opératoire pour 200g de la formulation suivante est le suivant :
• les pigments sont broyés 3 fois à la broyeuse tricylindre dans l'octyldodécanol porté au préalable à 60°C. Le broyat est laissé à refroidir à température ambiante (25°C) dans un poêlon double paroi ou un bécher.
• le copolymère , le squalane, le polybutylène, les nacres et le parfum sont ajoutés au broyat. Le tout est agité à la turbine (type : Rayneri) pour homogénéiser.
• quand le mélange est homogène, la polyphenyl trimethylsiloxy dimethylsiloxane est ajoutée sous agitation à 800 tours/minute au Rayneri pendant 30 minutes environ.
• Enfin, la sililce pyrogénée est ajoutée en pluie et l'agitation à la turbine est maintenue à 1000 tours/minute pendant 20 minutes.

| NOM | Concentration (% massique) |
|---|---|
| Perhydrosqualene végétal raffinée (Nom INCI = squalane) | 10,86 |
| Octyl-2 dodécanol | 15,39 |
| Oxyde de titane rutile traité aluminé/silice/trimethyolpropane | 2,74 |
| RED 7 | 0,54 |
| Lake Blue 1 | 0,16 |
| Lake Yellow 6 | 2,58 |
| Oxyde de fer noir | 0,25 |
| Mica - dioxyde de titan-e - oxyde de fer brun | 2 |
| Polyphenyltrimethylsiloxy dimethylsiloxane [1] | 20,03 |
| Silice pyrogénée hydrophobe, traitée en surface par diméthylsilane [3] | 4,5 |
| Poly(méthacrylate d'isobornyle-co-acrylate d'isobornyle-co-acrylate d'isobutyle-co-acide acrylique) à 50 % en matière active dans l'isododécane de l'exemple 1 | 30 |
| Polybutylène [2] | 10,65 |
| Parfum | 0,3 |
| Total | 100 |

[1] : Belsil PDM 1000 de WACKER (viscosité 1000 cPs PM : 9000)
[2] : INDOPOL H 100 (PM : 920)
[3] : AEROSIL R 972 de DEGUSSA

Cette composition de gloss, appliquée sur les lèvres en un seul geste, présente des propriétés de confort et de brillance satisfaisantes. La tenue de la composition est également améliorée ; la composition ne migre pas dans les rides et ridules du contour des lèvres.

## Revendications

1. Polymère séquencé, comprenant au moins une première séquence et au moins une deuxième séquence, **caractérisé en ce que** la première séquence est obtenue à partir d'au moins un monomère acrylate de formule $CH_2$ = CH-$COOR_2$ dans laquelle $R_2$ représente un groupe cycloalkyle $C_4$ à $C_{12}$ et d'au moins un monomère méthacrylate de formule $CH_2$ = C($CH_3$)-COOR'$_2$ dans laquelle R'$_2$ représente un groupe cycloalkyle $C_4$ à $C_{12}$, et

**caractérisé en ce que** la deuxième séquence est obtenue à partir d'un monomère acide acrylique et d'au moins un monomère de transition vitreuse inférieure ou égale à 20°C.

2. Polymère selon la revendication 1, **caractérisé en ce que** ledit polymère comprend une séquence intermédiaire comprenant au moins un monomère constitutif de la première séquence et au moins un monomère constitutif de la deuxième séquence.

3. Polymère selon la revendication 1 ou 2, **caractérisé en ce que** ledit polymère a un indice de polydispersité supérieur à 2.

4. Polymère selon l'une des revendications précédentes, **caractérisé en ce que** l'acrylate et le méthacrylate sont dans des propositions massiques comprises entre 30 :70 et 70 :30, de préférence entre 40 :50 et 50 :40 dans la première séquence.

5. Polymère selon l'une des revendications précédentes, **caractérisé en ce que** la première séquence est obtenue exclusivement à partir d'un monomère acrylate et d'un monomère méthacrylate.

6. Polymère selon l'une des revendications précédentes, **caractérisé en ce que** R2 et R'2 représentent un radical isobornyle.

7. Polymère selon l'une des revendications précédentes, **caractérisé en ce que** la proportion de la première séquence va de 20 à 90% en poids du polymère, mieux de 30 à 80% et encore mieux de 60 à 80%.

8. Polymère selon l'une des revendications précédentes, **caractérisé en ce que** ledit polymère contient de 1 à 10% en poids, de préférence de 3 à 8% de monomère acide acrylique.

9. Polymère selon l'une des revendications précédentes, **caractérisé en ce que** la deuxième séquence est obtenue à partir d'un monomère acide acrylique et d'un monomère acrylate de formule $CH_2 = CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S.

10. Polymère selon la revendication 9, **caractérisé en ce que** le monomère acide acrylique et le monomère acrylate sont dans des proportions massiques comprises entre 10 :90 et 20 :80, de préférence de l'ordre de 30 :70 dans la deuxième séquence.

11. Polymère selon la revendication 9 ou 10, **caractérisé en ce que** le monomère acrylate est l'acrylate d'isobutyle.

12. Polymère selon l'une des revendications précédentes, **caractérisé en ce que** la deuxième séquence est obtenue à partir d'acide acrylique et d'acrylate d'isobutyle et **en ce que** la première séquence est obtenue à partir d'acrylate d'isobornyle et de méthacrylate d'isobornyle.

13. Composition cosmétique **caractérisée en ce qu'**elle comprend un polymère selon l'une quelconque des revendications 1 à 12.

14. Composition cosmétique selon la revendication 13, **caractérisée en ce qu'**il s'agit d'une composition de maquillage ou de soin des matières kératiniques.

15. Composition cosmétique selon la revendication 13, **caractérisée en ce qu'**il s'agit d'un vernis à ongles.

16. Composition cosmétique selon la revendication 13, **caractérisée en ce qu'**il s'agit d'un produit de maquillage des lèvres.

17. Procédé cosmétique de maquillage ou de soin des matières kératiniques comprenant l'application sur les matières kératiniques d'une composition cosmétique selon l'une des revendications 13 à 16.

18. Utilisation d'un polymère selon l'une quelconque des revendications 1 à 12, dans une composition cosmétique, comme agent pour améliorer la tenue de ladite composition tout en maintenant sa brillance.

**19.** Procédé de préparation d'un polymère, consistant à mélanger, dans un même réacteur, un solvant de polymérisation, un amorceur, un monomère acide acrylique, au moins un monomère de transition vitreuse inférieure ou égale à 20°C, au moins un monomère acrylate de formule $CH_2$ = CH-$COOR_2$ dans laquelle $R_2$ représente un groupe cycloalkyle $C_4$ à $C_{12}$, et au moins un monomère méthacrylate de formule $CH_2$ = C($CH_3$)-COOR'$_2$ dans laquelle R'$_2$ représente un groupe cycloalkyle $C_4$ à $C_{12}$, selon la séquence d'étape suivante :

- On verse dans le réacteur, une partie du solvant de polymérisation et une partie de l'amorceur, mélange que l'on chauffe à une température de réaction comprise entre 60 et 120°C,
- on verse ensuite, en une première coulée, ledit au moins monomère acrylate de formule $CH_2$ = CH-$COOR_2$ et ledit au moins monomère méthacrylate de formule $CH_2$ = C($CH_3$)-$COOR_2$ que l'on laisse à réagir pendant une durée T correspondant à un taux de conversion desdits monomères de 90% maximum,
- on verse ensuite dans le réacteur, en une deuxième coulée, à nouveau de l'amorceur de polymérisation, le monomère acide acrylique et ledit au moins monomère de transition vitreuse inférieure ou égale à 20°C, qu'on laisse réagir pendant une durée T' au bout de laquelle le taux de conversion desdits monomères atteint un plateau,
- on ramène le mélange réactionnel à température ambiante.

**20.** Procédé selon la revendication 19, **caractérisé en ce que** la température de réaction est de l'ordre de 90 °C.

**21.** Procédé selon lune des revendications 19 ou 20, **caractérisé en ce que** le ratio massique entre le solvant de polymérisation et le polymère obtenu est compris entre 40 et 60% à la fin de la réaction.

**22.** Procédé selon lune des revendications 19 ou 21, **caractérisé en ce que** l'amorceur de polymérisaiton est un peroxyde.

**23.** Procédé selon l'une des revendications 19 à 22, **caractérisé en ce que** ledit au moins monomère acrylate de formule $CH_2$ = CH-$COOR_2$ et ledit au moins monomère méthacrylate de formule $CH_2$ = C($CH_3$)-$COOR_2$ sont versés dans le réacteur en deux fois : avec le solvant de polymérisation et pendant la première coulée.

**24.** Procédé selon l'une des revendications 19 à 23, **caractérisé en ce que** l'on verse du solvant de polymérisation pendant la première coulée.

**25.** Procédé selon l'une des revendications 19 à 24, **caractérisé en ce que** l'on verse du solvant de polymérisation pendant la deuxième coulée.

**26.** Procédé selon l'une des revendications 19 à 25, **caractérisé en ce que** l'acrylate de formule $CH_2$ = CH-$COOR_2$ et le méthacrylate $CH_2$ = C($CH_3$)-$COOR_2$ sont dans des propositions massiques comprises entre 30 :70 et 70 :30, de préférence entre 40 :50 et 50 :40.

**27.** Procédé selon l'une des revendications 19 à 26, **caractérisé en ce que** R2 représente un radical isobornyle.

**28.** Procédé selon l'une des revendications 19 à 27, **caractérisé en ce que** la proportion de l'acrylate de formule $CH_2$ = CH-$COOR_2$ et du méthacrylate $CH_2$ = C($CH_3$)-$COOR_2$ va de 20 à 90% en poids du polymère, mieux de 30 à 80% et encore mieux de 60 à 80%.

**29.** Procédé selon l'une des revendications 19 à 28, **caractérisé en ce que** l'acide acrylique représente de 1 à 10% en poids, de préférence de 3 à 8% du poids de tous les monomères introduits dans le réacteur.

**30.** Procédé selon l'une des revendications 19 à 29, **caractérisé en ce que** le monomère de transition vitreuse inférieure ou égale à 20°C est un monomère acrylate de formule $CH_2$ = $CHCOOR_3$, $R_3$ représentant une groupe alkyle non substitué en $C_1$ à $C_{12}$. linéaire ou ramifié, à l'exception du groupe tertiobutyle, dans lequel se trouve(nt) éventuellement intercalé(s) un ou plusieurs hétéroatomes choisis parmi O, N, S.

**31.** Procédé selon l'une des revendications 19 à 30, **caractérisé en ce que** le monomère acide acrylique et le monomère de transition vitreuse inférieure ou égale à 20°C sont dans des proportions massiques comprises entre 10 :90 et 20 :80, de préférence de l'ordre de 30 :70.

**32.** Procédé selon l'une des revendications 19 à 31, **caractérisé en ce que** le monomère de transition vitreuse inférieure

ou égale à 20°C est l'acrylate d'isobutyle.

**33.** Polymère susceptible d'être obtenu selon le procédé de l'une des revendications 19 à 33.

**34.** Composition cosmétique comprenant un polymère selon la revendication 33 ou un polymère obtenu selon le procédé de l'une des revendications 19 à 32.

## Claims

**1.** Block polymer comprising at least one first block and at least one second block,
**characterized in that** the first block is obtained from at least one acrylate monomer of formula $CH_2$=CH-COOR$_2$ in which $R_2$ represents a $C_4$ to $C_{12}$ cycloalkyl group and from at least one methacrylate monomer of formula $CH_2$=C($CH_3$)-COOR'$_2$ in which R'$_2$ represents a $C_4$ to $C_{12}$ cycloalkyl group,
and **characterized in that** the second block is obtained from an acrylic acid monomer and from at least one monomer with a glass transition of less than or equal to 20°C.

**2.** Polymer according to Claim 1, **characterized in that** the said polymer comprises an intermediate block comprising at least one constituent monomer of the first block and at least one constituent monomer of the second block.

**3.** Polymer according to Claim 1 or 2, **characterized in that** the said polymer has a polydispersity index of greater than 2.

**4.** Polymer according to one of the preceding claims,
**characterized in that** the acrylate and methacrylate are in proportions by weight of between 30:70 and 70:30, preferably between 40:50 and 50:40, in the first block.

**5.** Polymer according to one of the preceding claims,
**characterized in that** the first block is obtained exclusively from an acrylate monomer and from a methacrylate monomer.

**6.** Polymer according to one of the preceding claims,
**characterized in that** $R_2$ and R'$_2$ represent an isobornyl radical.

**7.** Polymer according to one of the preceding claims,
**characterized in that** the proportion of the first block ranges from 20 to 90% by weight of the polymer, better still from 30 to 80% and even better still from 60 to 80%.

**8.** Polymer according to one of the preceding claims,
**characterized in that** the said polymer comprises from 1 to 10% by weight, preferably from 3 to 8% by weight, of acrylic acid monomer.

**9.** Polymer according to one of the preceding claims,
**characterized in that** the second block is obtained from an acrylic acid monomer and from an acrylate monomer of formula $CH_2$=CHCOOR$_3$, $R_3$ representing a linear or branched unsubstituted $C_1$ to $C_{12}$ alkyl group, with the exception of the tert-butyl group, in which is/are optionally inserted one or more heteroatoms chosen from O, N and S.

**10.** Polymer according to Claim 9, **characterized in that** the acrylic acid monomer and the acrylate monomer are in proportions by weight of between 10:90 and 20:80, preferably of the order of 30:70, in the second block.

**11.** Polymer according to Claim 9 or 10, **characterized in that** the acrylate monomer is isobutyl acrylate.

**12.** Polymer according to one of the preceding claims,
**characterized in that** the second block is obtained from acrylic acid and from isobutyl acrylate and **in that** the first block is obtained from isobornyl acrylate and from isobornyl methacrylate.

**13.** Cosmetic composition, **characterized in that** it comprises a polymer according to any one of Claims 1 to 12.

**14.** Cosmetic composition according to Claim 13,

**characterized in that** it is a composition for making up or caring for keratinous substances.

**15.** Cosmetic composition according to Claim 13,
**characterized in that** it is a nail varnish.

**16.** Cosmetic composition according to Claim 13,
**characterized in that** it is a product for making up the lips.

**17.** Cosmetic process for making up or caring for keratinous substances, comprising application, to the keratinous substances, of a cosmetic composition according to one of Claims 13 to 16.

**18.** Use of a polymer according to any one of Claims 1 to 12 in a cosmetic composition as agent for improving the hold of the said composition while maintaining its gloss.

**19.** Process for the preparation of a polymer which consists in mixing, in the same reactor, a polymerization solvent, an initiator, an acrylic acid monomer, at least one monomer with a glass transition of less than or equal to 20°C, at least one acrylate monomer of formula $CH_2=CH-COOR_2$ in which $R_2$ represents a $C_4$ to $C_{12}$ cycloalkyl group and at least one methacrylate monomer of formula $CH_2=C(CH_3)-COOR'_2$ in which $R'_2$ represents a $C_4$ to $C_{12}$ cycloalkyl group, according to the following sequence of stages:

- a portion of the polymerization solvent and a portion of the initiator are run into the reactor, which mixture is heated to a reaction temperature of between 60 and 120°C,
- the said at least one acrylate monomer of formula $CH_2=CH-COOR_2$ and the said at least one methacrylate monomer of formula $CH_2=C(CH_3)-COOR'_2$ are subsequently run in, in a first fluid addition, and are left to react for a time T corresponding to a degree of conversion of the said monomers of at most 90%,
- subsequently, in a second fluid addition, again polymerization initiator, the acrylic acid monomer and the said at least one monomer with a glass transition of less than or equal to 20°C are run into the reactor and are left to react for a time T' at the end of which the degree of conversion of the said monomers reaches a plateau,
- the reaction mixture is brought back to ambient temperature.

**20.** Process according to Claim 19, **characterized in that** the reaction temperature is of the order of 90°C.

**21.** Process according to either of Claims 19 and 20,
**characterized in that** the ratio by weight of the polymerization solvent to the polymer obtained is between 40 and 60% at the end of the reaction.

**22.** Process according to either of Claims 19 and 21,
**characterized in that** the polymerization initiator is a peroxide.

**23.** Process according to one of Claims 19 to 22,
**characterized in that** the said at least one acrylate monomer of formula $CH_2=CH-COOR_2$ and the said at least one methacrylate monomer of formula $CH_2=C(CH_3)-COOR'_2$ are run into the reactor on two occasions: with the polymerization solvent and during the first fluid addition.

**24.** Process according to one of Claims 19 to 23,
**characterized in that** polymerization solvent is run in during the first fluid addition.

**25.** Process according to one of Claims 19 to 24,
**characterized in that** polymerization solvent is run in during the second fluid addition.

**26.** Process according to one of Claims 19 to 25,
**characterized in that** the acrylate of formula $CH_2=CH-COOR_2$ and the methacrylate $CH_2=C(CH_3)-COOR'_2$ are in proportions by weight of between 30:70 and 70:30, preferably between 40:50 and 50:40.

**27.** Process according to one of Claims 19 to 26,
**characterized in that** $R_2$ represents an isobornyl radical.

**28.** Process according to one of Claims 19 to 27,

**characterized in that** the proportion of the acrylate of formula $CH_2$=CH-COOR$_2$ and of the methacrylate $CH_2$=C($CH_3$)-COOR'$_2$ ranges from 20 to 90% by weight of the polymer, better still from 30 to 80% and even better still from 60 to 80%.

**29.** Process according to one of Claims 19 to 28,
**characterized in that** the acrylic acid represents from 1 to 10% by weight, preferably from 3 to 8% by weight, of the weight of all the monomers introduced into the reactor.

**30.** Process according to one of Claims 19 to 29,
**characterized in that** the monomer with a glass transition of less than or equal to 20°C is an acrylate monomer of formula $CH_2$=CHCOOR$_3$, $R_3$ representing a linear or branched unsubstituted $C_1$ to $C_{12}$ alkyl group, with the exception of the tert-butyl group, in which is/are optionally inserted one or more heteroatoms chosen from O, N and S.

**31.** Process according to one of Claims 19 to 30,
**characterized in that** the acrylic acid monomer and the monomer with a glass transition of less than or equal to 20°C are in proportions by weight of between 10:90 and 20:80, preferably of the order of 30:70.

**32.** Process according to one of Claims 19 to 31,
**characterized in that** the monomer with a glass transition of less than or equal to 20°C is isobutyl acrylate.

**33.** Polymer capable of being obtained according to the process of one of Claims 19 to 32.

**34.** Cosmetic composition comprising a polymer according to Claim 33 or a polymer obtained according to the process of one of Claims 19 to 32.

## Patentansprüche

1. Sequenzpolymer, das mindestens eine erste Sequenz und mindestens eine zweite Sequenz umfasst, **dadurch gekennzeichnet, dass** die erste Sequenz ausgehend von mindestens einem Acrylatmonomer der Formel $CH_2$ = CH-COOR$_2$, wobei $R_2$ eine $C_{4-12}$-Cycloalkylgruppe bedeutet, und mindestens einem Methacrylatmonomer der Formel $CH_2$ = C($CH_3$) -COOR'$_2$ gebildet wird, wobei R'$_2$ eine $C_{4-12}$-Cycloalkylgruppe bedeutet, und
**dadurch gekennzeichnet, dass** die zweite Sequenz ausgehend von einem Acrylsäuremonomer und mindestens einem Monomer mit einer Glasübergangstemperatur von 20 °C oder darunter gebildet wird.

2. Polymer nach Anspruch 1, **dadurch gekennzeichnet, dass** das Polymer eine Zwischensequenz aufweist, die mindestens ein die erste Sequenz aufbauendes Monomer und mindestens ein die zweite Sequenz aufbauendes Monomer umfasst.

3. Polymer nach Anspruch 1 oder 2, **dadurch gekennzeichnet, dass** das Polymer einen Polydispersitätsindex über 2 aufweist.

4. Polymer nach einem der vorhergehenden Ansprüche, **dadurch gekennzeichnet, dass** in der ersten Sequenz das Acrylat und das Methacrylat in Masseverhältnissen im Bereich von 30:70 bis 70:30 und vorzugsweise 40:50 bis 50:40 vorliegen.

5. Polymer nach einem der vorhergehenden Ansprüche, **dadurch gekennzeichnet, dass** die erste Sequenz ausschließlich ausgehend von einem Acrylatmonomer und einem Methacrylatmonomer gebildet wird.

6. Polymer nach einem der vorhergehenden Ansprüche, **dadurch gekennzeichnet, dass** $R_2$ und R'$_2$ die Isobornylgruppe bedeuten.

7. Polymer nach einem der vorhergehenden Ansprüche, **dadurch gekennzeichnet, dass** der Mengenanteil der ersten Polymersequenz im Bereich von 20 bis 90 Gew.-% des Polymers, besser 30 bis 80 % und noch besser 60 bis 80 % liegt.

8. Polymer nach einem der vorhergehenden Ansprüche, **dadurch gekennzeichnet, dass** das Polymer 1 bis 10 Gew.-% und vorzugsweise 3 bis 8 Gew.-% Acrylsäuremonomer enthält.

**9.** Polymer nach einem der vorhergehenden Ansprüche, **dadurch gekennzeichnet, dass** die zweite Sequenz ausgehend von einem Acrylsäuremonomer und einem Acrylatmonomer der Formel $CH_2$ = CH-$COOR_3$ gebildet wird, wobei die Gruppe $R_3$ mit Ausnahme der *tert*-Butylgruppe eine unsubstituierte, geradkettige und verzweigte $C_{1-12}$-Alkylgruppe bedeutet, in die gegebenenfalls ein oder mehrere Heteroatome eingebaut sind, die unter O, N, S ausgewählt sind.

**10.** Polymer nach Anspruch 9, **dadurch gekennzeichnet, dass** das Acrylsäuremonomer und das Acrylatmonomer in Masseanteilen im Bereich von 10:90 bis 20:80 und vorzugsweise in der Größenordnung von 30:70 in der zweiten Sequenz enthalten sind.

**11.** Polymer nach Anspruch 9 oder 10, **dadurch gekennzeichnet, dass** es sich bei dem Acrylmonomer um Isobutylacrylat handelt.

**12.** Polymer nach einem der vorhergehenden Ansprüche, **dadurch gekennzeichnet, dass** die zweite Sequenz ausgehend von Acrylsäure und Isobutylacrylat gebildet wird und **dadurch**, dass die erste Sequenz ausgehend von Isobornylacrylat und Isobornylmethacrylat erhalten wird.

**13.** Kosmetische Zusammensetzung, **dadurch gekennzeichnet, dass** sie ein Polymer nach einem der Ansprüche 1 bis 12 enthält.

**14.** Kosmetische Zusammensetzung nach Anspruch 13, **dadurch gekennzeichnet, dass** es sich um eine Zusammensetzung zum Schminken oder für die Pflege von Keratinsubstanzen handelt.

**15.** Kosmetische Zusammensetzung nach Anspruch 13, **dadurch gekennzeichnet, dass** es sich um einen Nagellack handelt.

**16.** Kosmetische Zusammensetzung nach Anspruch 13, **dadurch gekennzeichnet, dass** es sich um ein Produkt zum Schminken der Lippen handelt.

**17.** Kosmetisches Verfahren zum Schminken oder für die Pflege von Keratinsubstanzen, das umfasst, eine kosmetische Zusammensetzung nach einem der Ansprüche 13 bis 16 auf die Keratinsubstanzen aufzutragen.

**18.** Verwendung eines Polymers nach einem der Ansprüche 1 bis 12 in einer kosmetischen Zusammensetzung als Stoff zur Verbesserung der Haftung der Zusammensetzung, wobei gleichzeitig ihr Glanz erhalten bleibt.

**19.** Verfahren zur Herstellung eines Polymers, das darin besteht, in einem einzigen Reaktionsgefäß ein Polymerisationslösemittel, einen Initiator, ein Acrylsäuremonomer, mindestens ein Monomer mit einer Glasübergangstemperatur von höchstens 20 °C, mindestens ein Acrylatmonomer der Formel $CH_2$ = CH-$COOR_2$, wobei $R_2$ eine $C_{4-12}$-Cycloalkylgruppe bedeutet, und mindestens ein Methacrylatmonomer der Formel $CH_2$ = $C(CH_3)$ -$COOR'_2$, wobei $R'_2$ eine $C_{4-12}$-Cycloalkylgruppe bedeutet, gemäß den folgenden aufeinanderfolgenden Schritten zu vermischen:

man gibt einen Teil des Polymerisatiorlslösemittels und einen Teil des Initiators in das Reaktionsgefäß, wobei das Gemisch auf eine Reaktionstemperatur im Bereich von 60 bis 120 °C erwärmt wird;
dann fügt man in einer ersten Zugabe das mindestens eine Acrylatmonomer der Formel $CH_2$ = CH-$COOR_2$ und das mindestens eine Methacrylatmonomer der Formel $CH_2$ = $C(CH_3)$ -$COOR_2$ zu und lässt während einer Zeitdauer T reagieren, die einem Umwandlungsgrad der Monomere von 90 % maximal entspricht,
dann gibt man in den Reaktor in einer zweiten Zugabe nochmals Polymerisationsinitiator, das Acrylsäuremonomer und das mindestens eine Monomer mit einer Glasübergangstemperatur von höchstens 20 °C, wobei man während einer Zeitdauer T' reagieren lässt, nach der der Umwandlungsgrad der Monomere ein Plateau erreicht hat, das Reaktionsgemisch wird auf Raumtemperatur gebracht.

**20.** Verfahren nach Anspruch 19, **dadurch gekennzeichnet, dass** die Reaktionstemperatur in der Größenordnung von 90 °C liegt.

**21.** Verfahren nach einem der Ansprüche 19 oder 20, **dadurch gekennzeichnet, dass** das Masseverhältnis des Polymerisationslösemittels und des erhaltenen Polymeres am Ende der Reaktion im Bereich von 40 bis 60 % liegt.

**22.** Verfahren nach einem der Ansprüche 19 oder 21, **dadurch gekennzeichnet, dass** der Polymerisationsinitiator ein

Peroxid ist.

23. Verfahren nach einem der Ansprüche 19 bis 22, **dadurch gekennzeichnet, dass** das mindestens eine Acrylatmonomer der Formel $CH_2 = CH\text{-}COOR_2$ und das mindestens eine Methacrylatmonomer der Formel $CH_2 = C(CH_3)$ $\text{-}COOR_2$ in zwei Schritten in das Reaktionsgefäß gegeben werden: mit dem Polymerisationslösemittel und während der ersten Zugabe.

24. Verfahren nach einem der Ansprüche 19 bis 23, **dadurch gekennzeichnet, dass** während der ersten Zugabe Polymerisationslösemittel zugefügt wird.

25. Verfahren nach einem der Ansprüche 19 bis 24, **dadurch gekennzeichnet, dass** während der zweiten Zugabe Polymerisationslösemittel zugefügt wird.

26. Verfahren nach einem der Ansprüche 19 bis 25, **dadurch gekennzeichnet, dass** das Acrylat der Formel $CH_2 = CH\text{-}COOR_2$ und das Methacrylat der Formel $CH_2 = C(CH_3)\ \text{-}COOR_2$ in Masseverhältnissen im Bereich von 30:70 bis 70:30 und vorzugsweise 40:50 bis 50:40 zugegeben werden.

27. Verfahren nach einem der Ansprüche 19 bis 26, **dadurch gekennzeichnet, dass** die Gruppe $R_2$ die Isobornylgruppe bedeutet.

28. Verfahren nach einem der Ansprüche 19 bis 27, **dadurch gekennzeichnet, dass** der Anteil des Acrylats der Formel $CH_2 = CH\text{-}COOR_2$ und das Methacrylats der Formel $CH_2 = C(CH_3)\ \text{-}\ COOR_2$ im Bereich von 20 bis 90 Gew.-% des Polymers, besser 30 bis 80 % und noch besser 60 bis 80 % liegt.

29. Verfahren nach einem der Ansprüche 19 bis 28, **dadurch gekennzeichnet, dass** die Acrylsäure 1 bis 10 Gew.-% und vorzugsweise 3 bis 8 % des Gewichts aller in das Reaktionsgefäß eingebrachter Monomere ausmacht.

30. Verfahren nach einem der Ansprüche 19 bis 29, **dadurch gekennzeichnet, dass** das Monomer mit einer Glasübergangstemperatur von 20 °C oder darunter ein Acrylatmonomer der Formel $CH_2 = CH\text{-}COOR_3$ ist, wobei $R_3$ mit Ausnahme der *tert*-Butylgruppe eine unsubstituierte, geradkettige oder verzweigte $C_{1\text{-}12}$-Alkylgruppe bedeutet, in die gegebenenfalls ein oder mehrere Heteroatome eingebaut sind, die unter O, N, S ausgewählt sind.

31. Verfahren nach einem der Ansprüche 19 bis 30, **dadurch gekennzeichnet, dass** das Acrylsäuremonomer und das Monomer mit einer Glasübergangstemperatur von höchstens 20 °C in Masseanteilen von 10:90 bis 20:80 und vorzugsweise in der Größenordnung von 30:70 vorliegen.

32. Verfahren nach einem der Ansprüche 19 bis 31, **dadurch gekennzeichnet, dass** es sich bei dem Monomer mit einer Glasübergangstemperatur von höchstens 20 °C um Isobutylacrylat handelt.

33. Polymer, das nach dem Verfahren nach einem der Ansprüche 19 bis 33 erhältlich ist.

34. Kosmetische Zusammensetzung, die ein Polymer nach Anspruch 33 oder ein nach dem Verfahren gemäß einem der Ansprüche 19 bis 32 erhaltenen Polymer enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A


### Littérature non-brevet citée dans la description


- Polymer Handbook. John Wiley, 1989